# EUROPEAN PATENT SPECIFICATION

(11) **EP 2 009 118 B1**
(45) Date of publication and mention of the grant of the patent: **15.08.2012**
(21) Application number: 08466007.5
(22) Date of filing: 11.04.2008
(51) Int. Cl.: C12Q 1/68

(54) **Method for the detection and quantification of Mycobacterium avium subspecies paratuberculosis on the base of the polymerase chain reaction in the real time**
Verfahren zur Erkennung und Quantifizierung des Mycobakteriums Avium subspecies paratuberculosis auf Basis der Polymerkettenreaktion in Echtzeit
Procédé pour la détection et la quantification de Mycobacterium avium sous-espèce paratuberculosis sur la base de la réaction de chaîne polymérique en temps réel

(30) Priority: 23.04.2007 CZ 20070295
(43) Date of publication of application: 31.12.2008
(73) Proprietor: Vyzkumny ústav veterinárního lékarství, v.v.i., 621 32 Brno (CZ)
(72) Inventor: Slana, Iva, 618 00 Brno-Cernovice (CZ); Kralik, Petr, 612 00 Brno (CZ); Pavlik, Ivo, 602 00 Brno (CZ)
(74) Representative: Jirkalova, Hana

(56) References cited:
- HERTHNEK DAVID ET AL: "New PCR systems to confirm real-time PCR detection of Mycobacterium avium subsp. paratuberculosis" BMC MICROBIOLOGY, BIOMED CENTRAL, LONDON, GB, vol. 6, no. 1, 4 October 2006 (2006-10-04), page 87, XP021022432 ISSN: 1471-2180
- HRUSKA K., BARTOS M., KRALIK P., PAVLIK I.: "Mycobacterium avium subsp. paratuberculosis in powdered infant milk: paratuberculosis in cattle ? the public health problem to be solved" VET. MED. -CZECH, [Online] vol. 50, no. 8, 2005, pages 327-335, XP002504140 Retrieved from the Internet: URL:http://www.cazv.cz/attachments/VM_50-3 27-335.pdf> [retrieved on 2008-11-14]
- VANSNICK ELKE ET AL: "Newly developed primers for the detection of Mycobacterium avium subspecies paratuberculosis." VETERINARY MICROBIOLOGY 3 JUN 2004, vol. 100, no. 3-4, 3 June 2004 (2004-06-03), pages 197-204, XP002504141 ISSN: 0378-1135
- MCKENNA S L B ET AL: "Cow-level prevalence of paratuberculosis in culled dairy cows in Atlantic Canada and Maine." JOURNAL OF DAIRY SCIENCE NOV 2004, vol. 87, no. 11, November 2004 (2004-11), pages 3770-3777, XP002504142 ISSN: 0022-0302
- TASARA ET AL: "Development and evaluation of a Mycobacterium avium subspecies paratuberculosis (MAP) specific multiplex PCR assay" INTERNATIONAL JOURNAL OF FOOD MICROBIOLOGY, ELSEVIER SCIENCE PUBLISHERS, AMSTERDAM, NL, vol. 104, no. 3, 25 October 2005 (2005-10-25), pages 279-287, XP005112462 ISSN: 0168-1605
- DATABASE EBI 20 April 2007 (2007-04-20), "Mycobacterium avium subsp. paratuberculosis strain 39 insertion sequence IS900, partial sequence" XP002504146 Database accession no. EF536052
- TESINKA ET AL.: "Detection of the Mycobacterium avium subsp. paratuberculosis casual agent in milk by high sensitivity IS900 PCR and by quantitative IS900 and F57 real time PCR"[Online] 10 May 2007 (2007-05-10), - 13 May 2007 (2007-05-13) page 1, XP002504143 VENoMYC Athens Retrieved from the Internet: URL:http://www.ucm.es/info/venomyc/data/WP 5%20-%20WP8%20Workshop%20Athens%202007.pdf > [retrieved on 2008-11-14]
- WORLD ORGANISATION FOR ANIMAL HEALTH: "OIE Reference Laboratory Annual Report 2006"[Online] October 2007 (2007-10), pages 1-7, XP002504144 Retrieved from the Internet: URL:http://www.vri.cz/en/departments/food- and-feed-safety/oie-reference-laboratory-f or-paratuberculosis/> [retrieved on 2008-11-14]
- SLANA I, KRALIK P, KRALOVA A, PAVLIK I.: "On-farm spread of Mycobacterium avium subsp. paratuberculosis in raw milk studied by IS900 and F57 competitive real time quantitative PCR and culture examination." INT J FOOD MICROBIOL., 31 August 2008 (2008-08-31), pages 1-8, XP002504145

## Description

### Technical area

The invention is dealing with a method of detection and quantification of *Mycobacterium avium* subspecies *paratuberculosis (MAP)* by real-time polymerase chain reaction (real time PCR). Specifically, these are two independent real time PCR systems, which detect two loci IS*900* and *F57*, respectively specific for *MAP.*

### Present state of the technique

*MAP* is an intracellular parasitic bacterium, which is the causative agent of a chronic inflammatory disease of ruminant intestines designated as paratuberculosis or Johne's disease. The infected animals suffer from chronic diarrhoea and gradual weight loss. Paratuberculosis in ruminants is characterised by a long incubation time and a wide individual variability in pre-clinical and clinical phases of the disease development (Ayele et al., 2001).

In ruminants, *MAP* is above all shed through faeces, sperm and milk. *MAP* present in milk and milk products can become potential vectors of *MAP* to man and thus they are important from an aspect of food safety (Grant, 2006). Mycobacteria are relatively resistant to different temperatures and have been detected by culture in milk pasteurized at 71 and 72 °C (Ayele et al., 2005).

*MAP* is considered as one of the potential causative agents of Crohn's disease in humans, which is also manifested by inflammatory disease of the intestinal tract (Chamberlin et al., 2001; Bull et al., 2003). The exact aetiology of this disease remains obscure; however the model of autoimmune disease has been currently accepted. This model does not presume presence of live *MAP* in the patient's body. This may be the reason, why *MAP* was not found by culture in a number of patients with Crohn's disease, but only the below mentioned specific loci were detected (Baksh et al., 2004). From this aspect, children and immunocompromised patients are the most risky groups (Chamberlin et al., 2001; Hruska et al., 2005).

The *MAP* infections are diagnosed at present by a number of microbiological, immunological and molecular biology methods. The culture detection of the causative agent of paratuberculosis is considered as the "golden standard" at present. Long term *in vitro* growth of *MAP* (at least 2 to 3 months) is a disadvantage of this relatively reliable method. Indirect serological methods based on detection of antibodies against the causative agent of paratuberculosis specifically in the blood are specific and their sensitivity is low (Lombard et al., 2006). False negative results are often obtained or they cross-react with antibodies of genetically related mycobacteria from the environment. These results must be confirmed by direct detection of *MAP.*

Among the molecular biology methods, a number of systems based on conventional or "nested PCR" are used nowadays for direct detection of the causative agent of paratuberculosis. Two loci specific for *MAP* are most commonly used for the detection. Twelve to 18 copies of the insertion sequence IS*900* (X16293; Green et al., 1989) are found in the *MAP* genome (Bull et al., 2000) and it is considered as the "golden standard" for *MAP* detection by means of PCR. Only one copy of the genetic element *F57* (X70277) is present in the *MAP* genome (Poupart et al., 1993). Nevertheless, confirmation of the results obtained by PCR is necessary, especially if it is used for routine diagnosis. It is usually necessary to use hybridization of nucleic acids with specific probes, which however makes the whole process more demanding and applicable for experimental purposes only.

There are several published methods for the detection of *MAP* in different materials available (McKenna et al., 2004; Vasnick et al, 2004; Tasara et al., 2005) that use IS900 and F57 sequences as targets for the detection of MAP. These systems are based on the conventional or nested PCR and such approaches are required to have a kind of external control of PCR product specificity. It can be performed by e.g. hybridization with specific probe or by sequencing and/or PCR-RFLP analysis, which is time consuming and requires additional expenses (Rodriguez Lazaro et al., 2005).

The method of real time PCR has been recently employed for detection and potential quantification of *MAP* in biological material (Kim et al., 2002; O'Mahony and Hill, 2002; Herthnek and Bolske, 2006). It is based on visualization of the PCR products by means of fluorescent stains. High specificity and sensitivity are its benefits. To the best of our knowledge, two strategies of fluoresce labelling have been used: (1) incorporation of dsDNA specific fluorescent stain SYBR Green to the newly formed PCR products or (2) short oligonucleotides (hybridization probes) that are labelled with fluorescent stains and lie in the areas amplified by primers. Besides primers, they ensure the "second specificity" in real time PCR.

The first approach allows detection of *MAP* presence in the analysed material and determination of the specificity of the PCR product only according to the length of the PCR product based on the denaturation curve. Naturally, this assessment of specificity is not sequence specific and depends above all on the percentage of different nucleotides present in the resulting PCR product (O'Mahony and Hill, 2002; Rawa and Stanker, 2005). Hence, it is necessary to use additional hybridization in the checking for sequence specificity, when the internal amplification control (IAC) cannot be applied. Nowadays, the use of an IAC is necessary for diagnostic purposes, because it represents the only tool to differentiate the false negative from the truly negative samples. Provided that the sequence of the amplified segment is unique for *MAP,* the second approach allows qualitative and quantitative analysis without any need for further confirmation of specificity.

The most often used types of probes for *MAP* detection are: (1) hydrolysation probe and (2) FRET (fluorescence resonance energy transfer) probes.

When hydrolysation probes are used, these are digested by the exonuclease activity of DNA polymerase during amplification and fluorophor is released. Their advantage is that they are applicable with any real time PCR instrument available. On the other hand, FRET probes are based on simultaneous hybridization of two probes: one of them carries fluorophor at its 3' end (donor) and the second one is labelled with another fluorophor (acceptor) at 5' end. The donor energy emission is the accepted by acceptor and the wavelength of the latter is measured. The FRET probe is only applicable to Roche Diagnostics instruments (O'Mahony and Hill, 2004; Tasara and Stephan, 2005). The use of any probes labelled with a different fluorophore than is the probe for the target sequence allows IAC coamplification in one real time PCR reaction (Rodriguez Lazaro et al., 2005; Brey et al., 2006).

### The subject of invention

The above mentioned disadvantages are eliminated by the method of detection and quantification of *Mycobacterium avium* subspecies *paratuberculosis (MAP)* by two competitive real time polymerase chain reactions (PCR) each with its own internal amplification control, which is characterised by the fact that detection and quantification of *MAP* is performed by two independent real time PCR systems, which detect two loci specific for *MAP:* IS*900* and *F57*, respectively using primers and hydrolysation probes with the following sequences:
primers for *F57*

| | |
|---|---|
| F57qPCRF | GCCCATTTCATCGATACCC |

| | |
|---|---|
| F57qPCRR | GTACCGAATGTTGTTGTCAC, |

the probe for *F57*

| | |
|---|---|
| F57qPCRTM | FAM - CAATTCTCAGCTGCAACTCGAACACAC - BHQ, |

primers for IS*900*

| | |
|---|---|
| IS900qPCRF | GATGGCCGAAGGAGATTG |
| IS900qPCRR | CACAACCACCTCCGTAACC, |

the probe for IS*900*

| | |
|---|---|
| IS900qPCRTM | FAM - ATTGGATCGCTGTGTAAGGACACGT - BHQ, |

wherein the probe for the internal amplification controls has the following sequence

| | |
|---|---|
| IACqPCRTM | Cy5 - GGCTCTTCTATGTTCTGACCTTGTTGGA - BHQ. |

The method performed using primers and probes listed above is further characterised by the fact that the *MAP* assessment is performed in any sample of animal origin, eg. from farm animals, in clinical samples from human patients, samples of vegetable origin, samples from the environment, particularly fertilisers, dust and hay, samples of archaeological findings, or samples of food and feed.

The kits for detection and diagnosis of *MAP* with the method according to the invention are also a part of the subject of invention; they are characterised by the content of all oligonucleotide primers and probes, which are used for performing the method according to the invention.

The method according to the invention is dealing with two independent real time PCR systems for detection and quantification of *MAP* in biological material. They are based on amplification of *MAP* specific loci. The first one is based on amplification of the insertion sequence IS*900* and the second one on the amplification of the genetic element *F57.* Every sample is analysed by both systems. The advantage is that positivity of each sample is confirmed by two independent reactions and thus the risk of potential false positivity is excluded. The IS*900* real time PCR is more sensitive; however, it does not allow exact quantification of *MAP* in the analyzed samples. On the other hand, only one single copy of the genetic element *F57* is present in the *MAP* genome and therefore it is suitable for accurate quantification of *MAP* cells in samples.

Both real time PCR systems are based on the use of hydrolysation probes labelled with FAM (6-carboxyfluorescein) and thus they can be used for all available real time PCR instruments without the need for further confirmation of specificity.

Both systems include their own plasmid IAC that is based on competitive PCR (sequence of primers for the target sequence IS*900* or *F57* are identical with corresponding IAC). The use of only one set of primers for amplification of two PCR products in one real time PCR reaction reduces the risk of formation of nonspecific PCR products and consequent decrease in effectiveness of the PCR technique in the analysis of real samples. The inner sequences of both IAC are identical and that allows using only one Cy5-labelled probe for IAC in both PCR systems. Using only one set of primers for every real time PCR reaction and one probe for IAC reduces total financial costs of chemicals needed for the analysis.

The analysis protocol is identical for both real time PCR systems. It is an advantage in the case that few samples are processed. In one experiment, the same samples can be simultaneously analysed by both real time PCR systems, and thus the financial costs and time needed for analysis are notably reduced.

The standards for both real time PCR systems were prepared by cloning PCR products for IS900 and F57 into plasmid vector. The isolated plasmid DNA was diluted in the range between 1x10⁵ and 1x10⁰ plasmid copies per 1 µl of reaction. The appropriate dilution series was amplified in every experiment. It was used as the positive control of amplification and data source for construction of the calibration curve and thereby for determination of PCR efficiency of plasmid gradient calculated according to the regression curve equation. The calibration curve was used for exact quantification of *MAP* in unknown samples.

The method according to the invention, based on amplification of two loci specific for *MAP* using real time PCR, has been optimized for routine diagnosis of *MAP* in different types of biological material. The main advantage is the universal use (application in different laboratories and different instruments is possible without need for further modifications), it is not time-demanding and the low costs are combined with high specificity; sensitivity and reproducibility.

The following examples of performing the method according to the invention will document the method.

### Examples of performing the method

### Example 1

The method according to the invention consists of several steps:
**DNA material.** DNA templates from 17 bacterial and 4 mammalian species were used for confirmation of specificity of the developed real time PCR systems (Table 1).

Milk samples from 71 cows from a herd infected by paratuberculosis were used for testing the DNA isolation and the developed real time PCR systems. At the beginning of milking, the first milk ejections from every cow (the first 3 to 4 ejections from each of the teats that are usually discharged) were collected into a sterile disposable plastic container with a volume capacity of 200 ml. The milk samples were put into refrigerating boxes and delivered to the laboratories where they were stored at 4°C and processed within 3 days. Two independent samples from each sampling time were processed: one was used for DNA isolation and the other one as a stock sample for a case if DNA isolation or real time PCR failed.

**Design of primers and probes.** Primers and probes for insertion sequence IS*900* (X16293) and fragment *F57* (X70277) were designed by programme Primer3: http://frodo.wi.mit.edu/cgi-bin/primer3/primer3_www.cgi, (Table 2).

**Preparation of plasmid standards.** For preparation of plasmid standards for IS*900* and *F57* real time PCR, the PCR products of IS*900* and *F57* were amplified by conventional PCR using HotStar PCR Master Mix Kit (Qiagen, Hilden, Germany), 10 pmol of primers IS900qPCRF and IS900QPCRR or F57qPCRF and F57qPCRR and 2 µl of lysed *MAP* suspension (Collection strain CAPM 6381; Collection of Animal Pathogenic Microorganisms, Brno; http://www.vri.cz/labs/patogen/default.htm) in a total volume of 20 µl. PCR amplification was run under the following conditions: initial denaturation at 95°C for 15 min, followed by 30 cycles of denaturation at 95°C for 20 s, annealing at 60°C for 30 s, extension at 72°C for 1 min and final extension at 72°C for 5 min. Without further purification, the obtained PCR products were cloned into the vector pCR 2.1 (Invitrogen, Carlsbad, USA) according to the manufacturer's instructions.

Plasmids containing the inserts IS*900* (Tuor) and F57 (Beren) were purified using the Kit QIAprep Spin Miniprep Kit (Qiagen, Hilden, Germany), eluted in 200 µl TE buffer (Amresco, Inc, Solon, USA) and sequenced for confirmation of correct sequence of the insert. Exact concentration of the plasmid DNA and purity were assessed in triplicates in the spectrophotometer BioPhotometer (Eppendorf, Hamburg, Germany). The mean concentration value was used in the subsequent work with plasmid DNA. Simultaneously, the quality of plasmid DNA was visually checked by agarose gel electrophoresis. To increase the stability of plasmids in TE buffer during storage at -20°C and to decrease their loss during subsequent handling, 10 µg fish sperm DNA was added (Serva, Heidelberg, Germany). The resulting concentration of fish sperm DNA was 50 ng/µl.

**Preparation of internal amplification controls**. IAC for IS*900* and *F57* real time PCR are based on the principle of competitive PCR. IS*900* and *F57* "forward" and "reverse" primers have been fused at 3' ends with short oligonucleotides (TGTTAGAGAGG and ACTCTAAACCCAA) from the potato gene *St*TS1 (*Solanum tuberosum* Trehalose synthetase 1; AF483209). Both PCR products were prepared from potato DNA according to the above mentioned PCR protocol using the annealing temperature reduced to 50°C. Subsequently, they were processed identically as the plasmid standards for IS*900* and *F57*. The IAC plasmids for IS*900* (Idril) and *F57* (Luthien) were stored in TE buffer (Amresco, Solon, USA) at -20°C before further use.

**IS*900* and F57 real time PCR**. The conditions for IS*900* and *F57* real time PCR were optimized until the best concentration of primers and probes, concentration of IAC, concentration of MgCl₂ and conditions of the PCR protocol were determined. The optimized PCR reaction mixture for both real time PCR systems consisted of 1x DyNAmo Probe qPCR Kit (Finnzyme, Espoo, Finland), 10 pmol of primers IS900qPCRF and IS900QPCRR or F57qPCRF and F57qPCRR (final concentration 0.5 µM), 1 pmol of probes IS*900*qPCRTM or F57qPCRTM labelled with FAM (final concentration 0.05 µM), 4 pmol of Cy5-labelled probe for IAC IACQPCRTM (final concentration 0.2 µM), 0.2 U of Uracil DNA Glycosylase (Sigma, St. Louis, USA), 50 copies of plasmid Luthien or Idril and 5 µl of DNA template in a total volume of 20 µl.

Amplification and detection of fluorescence, which was identical for both real time PCR systems, was performed in the LightCycler 480 Instrument (Roche Molecular Diagnostic, Germany) in 96-well PCR plates under the following conditions: 37°C for 10 min, followed by initial denaturation at 95°C for 15 min and 47 cycles at 95°C for 5 s and 60°C for 40 s (fluorescence scanning). The subsequent analysis was conducted using the Fit point analysis in the software for LightCycler 480 (version 1.2.0.0625).

**Specificity of IS*900* and *F57* real time PCR**. Real time PCR experiments with lysed bacterial suspensions and DNA isolated from the peripheral blood of selected mammals were conducted to check specificity of the designed primers and probes for IS*900* and *F57* real time PCR (Table 1). One bacterial colony was resuspended in 20 µl of distilled water and denatured in a hot block at 100°C for 20 min. Subsequently, the produced suspension was centrifuged at 14 000 g for 10 min and the supernatant was used as a DNA template for real time PCR experiments. Mammalian DNA was isolated from peripheral blood using QIAamp DNA Blood Mini Kit (Qiagen, Hilden, Germany) according to the manufacturer's instructions.

**Sensitivity and reproducibility of IS*900* and *F57* real time PCR.** Sensitivity of the IS*900* and *F57* real time PCR systems was assessed by decimal dilution series of plasmids Tuor and Beren. Both plasmid gradients were diluted in the range between 10⁵ and 10⁰ copies/µl with TE buffer (Amresco, Inc, Solon, USA) containing 50 ng/µl fish sperm DNA (Serva, Heidelberg, Germany) and 10 copies/µl of plasmids Luthien and Idril. The prepared plasmid gradients were used for quantitative detections or as positive control in real time PCR and for calculation of the efficiency of the plasmid gradient amplification in the current PCR experiment. Therefore, an appropriate plasmid gradient was amplified in every real time PCR experiment. Reproducibility was determined by performing 20 independent repeated detections with freshly prepared dilutions of plasmid gradient.

**Isolation of total DNA from milk.** The initial 50 ml volume of fresh milk was centrifuged at 4 211 g (RCF) for 45 min in Hermle Z 383 K centrifuge (Hermle, Gosheim, Germany). The majority of the supernatant and cream was discharged. The pellet was resuspended in the remaining supernatant and transferred into another 2 ml tube. Subsequently, the samples were centrifuged at 14 000 g (RCF) for 10 min. The volume in every test tube was adjusted to 400 µl. Such "preprocessed samples" were stored at -80°C before use. The total milk DNA was isolated according to the modified protocol of the QIAamp DNA Blood Mini Kit (Qiagen, Hilden, Germany). After 5-min incubation, DNA was eluted in 50 µl of preheated TE buffer (Amresco, Inc, Solon, USA). The elution was performed twice. The isolation resulted in obtaining 100 µl of total DNA from milk in TE buffer containing carrier fish sperm DNA in concentration of 50 ng/µl. According to the parameters of the DNA isolation procedure, calculation of the number of *MAP* cells per 1 ml of initial milk volume was performed as follows: the number of F57 copies obtained in real time PCR was divided by 2 = number of *MAP* cells in 1 ml of initial milk volume. In the event of IS*900* real time PCR, the calculation was identical and moreover, the obtained number was divided by eighteen (1 *MAP* cell does not contain more than 18 copies of IS900; Bull et al., 2000).

**Measures for prevention of cross contamination.** Due to the fact that real time PCR is highly sensitive, the measures for prevention of cross contamination had to be strictly observed. DNA isolation, preparation of mixtures for real time PCR and addition of DNA template were performed in separate rooms with air overpressure. All tools and pipettes were washed with DNA-Exitus (AppliChem, Darmstadt, Germany) before use. Only filter tips were used. Negative control of isolation (water) that allows monitoring of potential contamination of the used chemicals and plastic materials was included in each run of DNA isolation. The chemicals and plastic materials for real time PCR were checked for contamination by means of negative PCR control (water instead of DNA template), which was included in each real time PCR experiment.

**Statistical analysis.** The values of crossing points obtained by IS900 and F57 real time PCR were calculated per actual number of copies according to the appropriate standard curves. The calculated values from gradients of plasmids Tuor and Beren were grouped and compared to each other using programme the InStat (GraphPad, San Diego, USA). The data were tested for the homogeneity of the variance by the Kolmogorov and Smirnov test and the variance by Student's T-test.The groups of data were tested for normality of splitting and variance by means of a test of variability, i.e. Kolmogorov-Smirnov test and T-test, respectively. The differences at P < 0.05 were considered as significant. The coefficient of variability (CV) was calculated with the aim to measure accuracy of DNA isolation and subsequent real time PCR. For indication of the variance of respective calculated values, 95% confidence interval was determined.

### Results

**Optimisation of IS*900* and *F57* real time PCR**. Optimal amount of copies of the plasmids Luthien or Idril, which ensures optimal coamplification with the target sequence, was determined by titration of 5 x 10³, 5 x 10², 5 x 10¹ and 5 x 10⁰ of each IAC copy to PCR reaction and appropriate plasmid gradient. The results were identical for both real time PCR systems. It was found that 5 x 10¹ copies of the plasmid Idril or Luthien per reaction (values of crossing point between 35 and 36) did not affect amplification of appropriate plasmid gradient even in the lowest concentrations and provided a sufficiently strong signal that could be distinguished from the background.

In the next step, the effect of nonspecific fish sperm DNA present in a template on the amplification of a target molecule during real time PCR was investigated. Plasmids Idril and Luthien (10 copies/µl) were diluted in TE buffer containing 0, 10, 30, 50, 70, 90, 100 and 200 ng/µl fish sperm DNA and were used as diluents for preparation of plasmid gradients Tuor and Beren. Thus the prepared templates were analysed by IS*900* or *F57* based real time PCR in triplicates.

The values of "crossing points" of both gradients were not affected even by presence of 100 ng/µl (500 ng per PCR reaction) of nonspecific DNA. The concentration of 200 ng/µl significantly inhibited both real time PCR systems. For other experiments, the concentration of 50 ng/µl was used.

**Specificity of IS*900* and *F57* real time PCR**. Both real time PCR systems were tested for capability to selectively distinguish DNA of *MAP* from other bacterial lysates and mammalian DNA (Table 1). No bacterial or mammalian DNA except *MAP* was amplified by either *F57* or IS*900* real time PCR and all negative samples showed a clear signal for IAC. Mammalian species were chosen as the most likely sources of DNA background for the developed real time PCR systems.

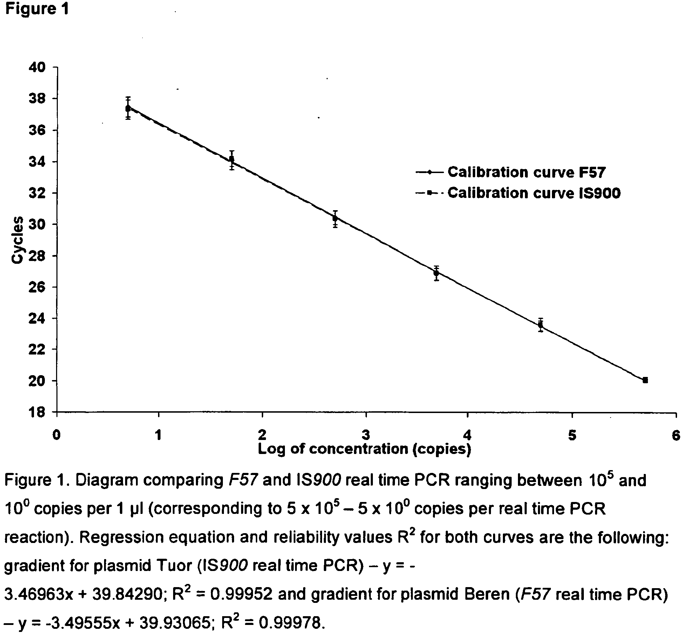

**Sensitivity of IS*900* and *F57* real time PCR**. By both real time PCR systems, one copy of plasmid Beren or Tuor per 1 µl (5 copies per reaction) can be detected in all 20 replicates (Table 3, Figure 1). Comparison of the calculated datasets for *F57* and IS*900* using T-test did not show a significant difference (P > 0.1) between them.

**Isolation of DNA from field samples of milk**. Both developed real time PCR systems were used for detection and quantification of *MAP* in 71 milk samples from a herd of cows infected with paratuberculosis (Table 4). The collected milk samples were tagged with codes, DNA was isolated from them and analyses by IS*900* and *F57* real time PCR were performed. Samples, which were slightly positive for IS*900* and negative for *F57*, were considered as positive. Every field sample was analysed in duplicate and was considered as positive only in the case that both duplicates were positive; otherwise DNA isolation was repeated and both real time PCR were performed using the reserve sample.

The proportion between the calculated IS*900* and *F57* copy numbers are presented in Table 4. The copy numbers of IS*900* were always higher than *F57* in all the samples with simultaneously positive results from IS*900* and *F57* real time PCR and the ratio 12 to 18:1 was detected in almost all samples. The copy number was not higher than 50 in all samples where IS*900* was only detected; that corresponded to approximately 3 *MAP* cells.

**Experimental limit of *MAP* detection in milk.** After calculation of the copy numbers obtained from real time PCR per initial volume of milk, the experimental limit of detection was determined: 1 *MAP* per 2 ml milk for IS*900* real time PCR and 2 *MAP* cells per 1 ml milk for *F57* real time PCR. We can suppose that in fact, no detection system is perfect. Despite all precautions, target sites may be lost during handling a sample due to either DNA fragmentation during isolation or adhesion to the consumable plastic material. Therefore a lower probability of detection in low concentrations should be expected. DNA extracted from a complex material such as milk may contain traces of inhibitory substances. These will not be reflected in IAC amplification, but they may inhibit the low concentrations of target sites (Herthnek and Bolske, 2006).

### Applicability for the industry

This method of detection and quantification of *MAP* is above all intended for routine diagnosis. It is designed for quantification of *MAP* in any biological material. The prerequisite of its successful use is isolation of high-quality DNA from a given matrix. The developed real time PCR systems were tested under practical conditions on DNA samples isolated from milk, which can be the primary source of *MAP* transmission to man.

The above mentioned method comprises the real time PCR protocol with hydrolysis probes and IAC, including PCR mix composition and proportions in different components, PCR protocol, the analysis of obtained data and quantification of *MAP* in samples.

### REFERENCES

Ayele, W.Y., Machackova, M., Pavlik, I. (2001): The transmission and impact of paratuberculosis infection in domestic and wild ruminants. Veterinarni Medicina, 46, 205-224.
Ayele, W.Y., Svastova, P., Roubal, P., Bartos, M., Pavlik, I. (2005): Mycobacterium avium subspecies paratuberculosis cultured from locally and commercially pasteurized cow's milk in the Czech Republic. Appl.Environ.Microbiol., 71, 1210-1214.
Baksh, F.K., Finkelstein, S.D., riyanayagam-Baksh, S.M., Swalsky, P.A., Klein, E.C., Dunn, J.C. (2004): Absence of Mycobacterium avium subsp paratuberculosis in the microdissected granulomas of Crohn's disease. Modern Pathology, 17, 1289-1294.
Brey, B.J., Radcliff, R.P., Clark, D.L., Jr., Ellingson, J.L. (2006): Design and development of an internal control plasmid for the detection of Mycobacterium avium subsp. paratuberculosis using real-time PCR. Mol.Cell Probes, 20, 51-59.
Bull, T.J., Hermon-Taylor, J., Pavlik, I., EI Zaatari, F., Tizard, M. (2000): Characterization of IS900 loci in Mycobacterium avium subsp. paratuberculosis and development of multiplex PCR typing. Microbiology, 146 (Pt 9), 2185-2197.
Bull, T.J., McMinn, E.J., Sidi-Boumedine, K., Skull, A., Durkin, D., Neild, P., Rhodes, G., Pickup, R., Hermon-Taylor, J. (2003): Detection and verification of Mycobacterium avium subsp. paratuberculosis in fresh ileocolonic mucosal biopsy specimens from individuals with and without Crohn's disease. J.Clin.Microbiol., 41, 2915-2923.
Chamberlin, W., Graham, D.Y., Hulten, K., EI Zimaity, H.M., Schwartz, M.R., Naser, S., Shafran, I., EI Zaatari, F.A. (2001): Review article: Mycobacterium avium subsp. paratuberculosis as one cause of Crohn's disease. Aliment.Pharmacol.Ther., 15, 337-346.
Grant, I.R. (2006): Mycobacterium avium ssp paratuberculosis in foods: current evidence and potential consequences. International Journal of Dairy Technology, 59, 112-117.
Green, E.P., Tizard, M.L., Moss, M.T., Thompson, J., Winterbourne, D.J., McFadden, J.J., Hermon-Taylor, J. (25-11-1989): Sequence and characteristics of IS900, an insertion element identified in a human Crohn's disease isolate of Mycobacterium paratuberculosis. Nucleic Acids Res., 17, 9063-9073.
Herthnek, D., Bolske, G. (2006): New PCR systems to confirm real-time PCR detection of Mycobacterium avium subsp paratuberculosis. Bmc Microbiology, 6,
Hruska, K., Bartos, M., Kralik, P., Pavlik, I. (2005): Mycobacterium avium subsp paratuberculosis in powdered infant milk: paratuberculosis in cattle - the public health problem to be solved. Veterinarni Medicina, 50, 327-335.
Kim, S.G., Shin, S.J., Jacobson, R.H., Miller, L.J., Harpending, P.R., Stehman, S.M., Rossiter, C.A., Lein, D.A. (2002): Development and application of quantitative polymerase chain reaction assay based on the ABI 7700 system (TaqMan) for detection and quantification of Mycobacterium avium subsp paratuberculosis. Journal of Veterinary Diagnostic Investigation, 14, 126-131.
Lombard, J.E., Byrem, T.M., Wagner, B.A., McCluskey, B.J. (2006): Comparison of milk and serum enzyme-linked immunosorbent assays for diagnosis of Mycobacterium avium subspecies paratuberculosis infection in dairy cattle. Journal of Veterinary Diagnostic Investigation, 18, 448-458.
McKenna, S.L., Keefe, G.P., Barkema, H.W., McClure, J., Vanleeuwen, J.A., Hanna, P., Sockett, D.C. (2004): Cow-level prevalence of paratuberculosis in culled dairy cows in Atlantic Canada and Maine. J.Dairy Sci., 87 (11), 3770-3777.
O'Mahony, J., Hill, C. (2002): A real time PCR assay for the detection and quantitation of Mycobacterium avium subsp. paratuberculosis using SYBR Green and the Light Cycler. J.Microbiol.Methods, 51, 283-293.
O'Mahony, J., Hill, C. (2004): Rapid real-time PCR assay for detection and quantitation of Mycobacterium avium subsp. paratuberculosis DNA in artificially contaminated milk. Appl.Environ.Microbiol., 70, 4561-4568.
Poupart, P., Coene, M., Van Heuverswyn, H., Cocito, C. (1993): Preparation of a specific RNA probe for detection of Mycobacterium paratuberculosis and diagnosis of Johne's disease. J.Clin.Microbiol., 31, 1601-1605.
Ravva, S.V., Stanker, L.H. (2005): Real-time quantitative PCR detection of Mycobacterium avium subsp. paratuberculosis and differentiation from other mycobacteria using SYBR Green and TaqMan assays. J.Microbiol.Methods, 63, 305-317.
Rodriguez-Lazaro, D., D'Agostino, M., Herrewegh, A., Pla, M., Cook, N., Ikonomopoulos, J. (1-5-2005): Real-time PCR-based methods for detection of Mycobacterium avium subsp. paratuberculosis in water and milk. Int.J.Food Microbiol., 101, 93-104.
Tasara, T., Hoelzle, L.E., Stephan, R. (2005): Development and evaluation of a Mycobacterium avium subspecies paratuberculosis (MAP) specific multiplex PCR assay. Int.J.Food Microbiol., 104 (3), 279-287.
Tasara, T., Stephan, R. (2005): Development of an F57 sequence-based real-time PCR assay for detection of Mycobacterium avium subsp. paratuberculosis in milk. Appl.Environ.Microbiol., 71, 5957-5968.
Vansnick, E., de Rijk, P., Vercammen, F., Geysen, D., Rigouts, L., Portaels, F. (2004): Newly developed primers for the detection of Mycobacterium avium subspecies paratuberculosis. Vet.Microbiol., 100 (3-4), 197-204.

**Table 1**

| List of bacterial and mammalian species tested by IS*900* and *F57* real time PCR with corresponding internal amplification control | | | |
|---|---|---|---|
| Species | Source | IS*900* | *F57* |
| *M. avium* subsp. *paratuberculosis* | CAPM^{a} 6381 | + | + |
| *M. avium* subsp. *paratuberculosis* | field sample (bear), CR | + | + |
| *M. avium* subsp. *paratuberculosis* | field sample (fallow deer), CR | + | + |
| *M. avium* subsp. *paratuberculosis* | field sample (faeces), CR | + | + |
| *M. avium* subsp. *paratuberculosis* | field sample (moufflon), CR | + | + |
| *M. avium* subsp. *paratuberculosis* | field sample (cattle), CR | + | + |
| *M. avium* subsp. *avium* | CAPM 5889 | - | - |
| *M. avium* subsp. *avium* | field sample (rabbit), CR | - | - |
| *M. avium* subsp. *hominisuis* | field sample (pig), CR | - | - |
| *M. avium* subsp. *hominisuis* | clinical isolate (man), CR | - | - |
| *M. gordonae* | ATCC*^{b}* 12478 | - | - |
| *M. cansasii* | ATCC 12478 | - | - |
| *M. scrofulaceum* | ATCC 19981 | - | - |
| *M. szulgai* | ATCC 35799 | - | - |
| *M. intracellulare* | CAPM 5627 | - | - |
| *Escherichia coli* | competent cells (Invitrogen) | - | - |
| *Salmonella enterica* serovar Typhimurium | CAPM 5438 | - | - |
| Sheep (*Ovis aries*) | field isolate DNA, CR | - | - |
| Goat (*Capra hircus*) | field isolate DNA, CR | - | - |
| Cattle (*Bos taurus*) | field isolate DNA, CR | - | - |
| Man (*Homo sapiens sapiens*) | clinical isolate, CR | - | - |

| | | | |
|---|---|---|---|
| *^{a}*Collection of Animal Pathogenic Microorganisms *^{b}*The American Type Culture Collection | | | |

**Table 2**

| Sequence of the used primers and probes*^{a}* | | | | | | |
|---|---|---|---|---|---|---|
| Target gene | Name | Type | Sequence 5'→3' | Localisation | Length of PCR product (bp) | Name of plasmid standard |
| | IS*900*qPCRF | Forward | GATGGCCGAAGGAGATTG | 422 - 440 | | |
| IS*900* | IS*900*qPCRR | Reverse | CACAACCACCTCCGTAACC | 568 - 549 | 145 | Tuor |
| | IS*900*qPCRTM | Probe | FAM - ATTGGATCGCTGTGTAAGGACACGT - BHQ | 508 - 534 | | |
| | F57qPCRF | Forward | GCCCATTTCATCGATACCC | 94 - 111 | | |
| *F57* | F57qPCRR | Reverse | GTACCGAATGTTGTTGTCAC | 238 - 220 | 147 | Beren |
| | F57qPCRTM | Probe | FAM – CAATTCTCAGCTGCAACTCGAACACAC – BHQ | 158 - 182 | | |
| IAC | IACqPCRTM | Probe | Cy5 – GGCTCTTCTATGTTCTGACCTTGTTGGA – BHQ | | IS*900* 152 | Luthien |
| | | | | | *F57* 154 | Idril |

| | | | | | | |
|---|---|---|---|---|---|---|
| *^{a}*All primers and probes were synthesized in VBC Biotech (Wien, Austria). | | | | | | |

**Table 3**

| | | | | | | | | | | |
|---|---|---|---|---|---|---|---|---|---|---|
| Evaluation of plasmid Beren and Tuor gradient by real time PCR method | | | | | | | | | | |

| Plasmid gradient | | CP^{a} | | Real number of copies*^{b}* | | | IS*^{c}* | | Ratio*^{d}* | Mean effectiveness of PCR*^{e}* |
|---|---|---|---|---|---|---|---|---|---|---|
| Designation | Number of copies per PCR reaction | Mean | SO*^{f}* | Mean | SO | CV*^{g}* | Upper | Lower | | |
| | 500000 | 20.05 | 0.07 | 516915 | 94880 | 18.36 | 557495 | 476335 | 20/20 | |
| Tuor (IS*900* real time PCR) | 50000 | 23.63 | 0.41 | 47441 | 6177 | 13.02 | 50083 | 44799 | 20/20 | |
| | 5000 | 26.91 | 0.47 | 5424 | 1139 | 21.00 | 5911 | 4937 | 20/20 | 94.18% |
| | 500 | 30.37 | 0.51 | 548 | 119 | 21.73 | 599 | 498 | 20/20 | |
| | 50 | 34.19 | 0.51 | 44 | 14 | 31.65 | 50 | 38 | 20/20 | |
| | 5 | 37.31 | 0.61 | 6 | 1 | 23.13 | 6 | 5 | 20/20 | |
| | 500000 | 20.09 | 0.16 | 479470 | 62799 | 13.10 | 506329 | 452611 | 20/20 | |
| Beren (*F57* real time PCR) | 50000 | 23.52 | 0.34 | 50009 | 7180 | 14.36 | 53080 | 46938 | 20/20 | |
| | 5000 | 26.84 | 0.37 | 5641 | 1186 | 21.03 | 6148 | 5134 | 20/20 | 93.23% |
| | 500 | 30.45 | 0.43 | 525 | 98 | 18.74 | 567 | 483 | 20/20 | |
| | 50 | 34.11 | 0.61 | 48 | 15 | 30.62 | 54 | 42 | 20/20 | |
| | 5 | 37.48 | 0.64 | 5 | 1 | 23.03 | 6 | 5 | 20/20 | |

| | | | | | | | | | | |
|---|---|---|---|---|---|---|---|---|---|---|
| *^{a}* CP - The "crossing point" value *^{b}* Calculation was performed from CP according to the regression equation for respective experiment and the means, SO and CV were subsequently calculated *^{c}* IS - Experimental 95% interval of reliability *^{d}* Positive samples/a total of samples *^{e}* Calculated from mean regression coefficient *^{f}* SO - Standard deviation *^{g}* CV - Coefficient of variance | | | | | | | | | | |

**Table 4**

| Detection of *MAP* in cow milk (the first ejections from all teats) from a herd of cows infected with paratuberculosis | | | | | | | |
|---|---|---|---|---|---|---|---|
| IS*900*+ and F57+ | | | | IS*900*+ and F57- | | | |
| Cow No. | IS*900^{a}* | *F57^{a}* | Number of *MAP^{b}* | Cow No. | IS*900^{a}* | *F57^{a}* | Number of *MAP^{c}* |
| 1 | 37 | 10 | 5 | 1 | 52 | 0 | <2 |
| 2 | 598 | 58 | 29 | 2 | 22 | 0 | <2 |
| 3 | 12 | 9 | 5 | 3 | 19 | 0 | <2 |
| 4 | 30 | 4 | 2 | 4 | 16 | 0 | <2 |
| 5 | 4 | 3 | 2 | 5 | 3 | 0 | <2 |
| 6 | 34 | 6 | 3 | 6 | 13 | 0 | <2 |
| 7 | 679 | 74 | 37 | 7 | 1 | 0 | <2 |
| 8 | 53 | 10 | 5 | 8 | 4 | 0 | <2 |
| 9 | 10 | 4 | 2 | 9 | 16 | 0 | <2 |
| 10 | 37 | 3 | 2 | 10 | 6 | 0 | <2 |
| 11 | 30 | 5 | 3 | 11 | 9 | 0 | <2 |
| 12 | 49 | 5 | 3 | 12 | 3 | 0 | <2 |
| 13 | 160 | 6 | 3 | 13 | 14 | 0 | <2 |
| 14 | 399 | 7 | 4 | 14 | 30 | 0 | <2 |
| 15 | 6 | 4 | 2 | 15 | 36 | 0 | <2 |
| | | | | 16 | 34 | 0 | <2 |
| | | | | 17 | 24 | 0 | <2 |
| | | | | 18 | 21 | 0 | <2 |
| | | | | 19 | 45 | 0 | <2 |
| | | | | 20 | 25 | 0 | <2 |
| | | | | 21 | 47 | 0 | <2 |
| | | | | 22 | 6 | 0 | <2 |

| | | | | | | | |
|---|---|---|---|---|---|---|---|
| ^{a} Mean number of copies from a duplicate per PCR reaction. ^{b} Number of *MAP* cells per 1 ml of initial milk volume. Calculated from the amount of *MAP* cells detected by *F57* real time PCR (number of *F57* copies obtained from real time PCR divided by 2 = number of *MAP* cells per 1 ml of initial milk volume). *^{c}*Number of *MAP* cells per 1 ml of initial milk volume. Calculated from the amount of *MAP* cells detected by IS*900* real time PCR (number of IS*900* copies obtained from real time PCR divided by 36 = number of *MAP* cells per 1 ml of initial milk volume).. | | | | | | | |

## Claims

1. A method for detection and quantification of *Mycobacterium avium* subspecies *paratuberculosis* by two competitive real time polymerase chain reactions each with its own internal amplification control, which is **characterised by** the fact that detection and quantification of *Mycobacterium avium* subspecies *paratuberculosis* is performed by two independent real time polymerase chain reactions systems, which detect two loci specific for *Mycobacterium avium* subspecies *paratuberculosis:* IS900 and *F57*, respectively, using primers and hydrolysation probes having the following sequences
primers for F57
| | |
|---|---|
| **F57qPCRF** | **GCCCATTTCATCGATACCC** |
| **F57qPCRR** | **GTACCGAATGTTGTTGTCAC,** |
the probe for F57
| | |
|---|---|
| **F57qPCRTM** | **FAM - CAATTCTCAGCTGCAACTCGAACACAC - BHQ,** |
primers for IS900
| | |
|---|---|
| **IS900qPCRF** | **GATGGCCGAAGGAGATTG** |
| **IS900qPCRR** | **CACAACCACCTCCGTAACC,** |
the probe for IS900
| | |
|---|---|
| **IS900qPCRTM** | **FAM - ATTGGATCGCTGTGTAAGGACACGT - BHQ,** |
wherein the probe for the internal amplification control has the following sequence
| | |
|---|---|
| **IACqPCRTM** | **Cy5 - GGCTCTTCTATGTTCTGACCTTGTTGGA - BHQ.** |

2. The method according to claim 1, **characterised by** the fact that *Mycobacterium avium* subspecies *paratuberculosis* is detected and quantified in any sample of animal origin, in clinical samples from human patients, in samples of vegetable origin, in samples from the environment, especially from fertilizers, dust and hay, in samples from archaeological findings or in food and feed samples.

3. A kit for detection and diagnosis of *Mycobacterium avium* subspecies *paratuberculosis* according to the method of claim 1, comprising all the following primers and probes, each of which consists of the sequence defined below:
primers for F57
| | |
|---|---|
| **F57qPCRF** | **GCCCATTTCATCGATACCC** |
| **F57qPCRR** | **GTACCGAATGTTGTTGTCAC,** |
the probe for F57
| | |
|---|---|
| **F57qPCRTM** | **FAM - CAATTCTCAGCTGCAACTCGAACACAC - BHQ,** |
primers for IS900
| | |
|---|---|
| **IS900qPCRF** | **GATGGCCGAAGGAGATTG** |
| **IS900qPCRR** | **CACAACCACCTCCGTAACC,** |
the probe for IS900
| | |
|---|---|
| **IS900qPCRTM** | **FAM - ATTGGATCGCTGTGTAAGGACACGT - BHQ,** |
and the probe for the internal amplification control
| | |
|---|---|
| **IACqPCRTM** | **Cy5 - GGCTCTTCTATGTTCTGACCTTGTTGGA - BHQ.** |

## Patentansprüche

1. Das Verfahren zum Nachweis und Quantifizierung von *Mycobacterium avium* subspecies *paratuberculosis* mit zwei kompetitiven Polymerase-Kettenreaktionen in Echtzeit, jede mit der eigenen internen Amplifikationskontrolle. Das Verfahren zeichnet sich dadurch aus, dass der Nachweis und Quantifizierung von *Mycobacterium avium* subspecies *paratuberculosis* mit zwei kompetitiven Polymerase-Kettenreaktionen in Echtzeit durchgeführt wird, die zwei Zielgene spezifisch für *Mycobacterium avium* subspecies *paratuberculosis* identifizieren können: IS*900* und *F57*, bei Verwendung von Primern und Hydrolysesonden mit den folgenden Sequenzen für:
die Primer für *F57*
| | |
|---|---|
| F57qPCRF | GCCCATTTCATCGATACCC |
| F57qPCRR | GTACCGAATGTTGTTGTCAC, |
die Sonde für *F57*
| | |
|---|---|
| F57qPCRTM | FAM - CAATTCTCAGCTGCAACTCGAACACAC - BHQ, |
die Primer für das IS*900*
| | |
|---|---|
| IS900qPCRF | GATGGCCGAAGGAGATTG |
| IS900qPCRR | CACAACCACCTCCGTAACC, |
die Sonde für das IS900
| | |
|---|---|
| IS900qPCRTM | FAM - ATTGGATCGCTGTGTAAGGACACGT - BHQ, |
wobei die Zusammensetzung der Sonde für die interne Amplifikationskontrolle ist:
| | |
|---|---|
| IACqPCRTM | Cy5 - GGCTCTTCTATGTTCTGACCTTGTTGGA - BHQ. |

2. Das Verfahren nach dem Anspruch 1, das sich dadurch auszeichnet, dass der Nachweis und Quantifizierung von *Mycobacterium avium* subspecies *paratuberculosis* ausgeführt werden kann in Proben von beliebigem Material tierischen wie auch pflanzlichen Ursprungs, in Lebens- und Futtermittelproben, in klinischen Proben bei Patienten in Humanmedizin, in Umweltproben, insbesondere von Düngemitteln, Staub, Heu oder in Proben aus archäologischen Funden.

3. Detektionsets zum Nachweis und Diagnostik von *Mycobacterium avium* subspecies *paratuberculosis* nach dem Anspruch 1, die alle folgenden Primer und Sonden einschließen, die aus unten angeführten Sequenzen bestehen:
die Primer für *F57*
| | |
|---|---|
| F57qPCRF | GCCCATTTCATCGATACCC |
| F57qPCRR | GTACCGAATGTTGTTGTCAC, |
die Sonde für *F57*
| | |
|---|---|
| F57qPCRTM | FAM - CAATTCTCAGCTGCAACTCGAACACAC - BHQ, |
die Primer für das IS*900*
| | |
|---|---|
| IS900qPCRF | GATGGCCGAAGGAGATTG |
| IS900qPCRR | CACAACCACCTCCGTAACC, |
die Sonde für das IS*900*
| | |
|---|---|
| IS900qPCRTM | FAM - ATTGGATCGCTGTGTAAGGACACGT - BHQ, |
und die Sonde für die interne Amplifikationskontrolle
| | |
|---|---|
| IACqPCRTM | Cy5 - GGCTCTTCTATGTTCTGACCTTGTTGGA - BHQ. |

## Revendications

1. Le mode de détection et de quantification du *Mycobacterium avium* sous espèce du *paratuberculosis* par deux réactions en chaine compétitive par polymérase en temps réel, chacune avec un propre contrôle d'amplification interne, **caractérisé par le fait que** la détection et la quantification du *Mycobacterium avium* sous-espèce du *paratuberculosis* sont effectuées par deux réactions en chaine par polymérase en temps réel qui détecte deux locus spécifiques pour le *Mycobacterium avium* sous-espèce du *paratuberculosis:* IS*900* et *F57,* en utilisant des amorces et les sondes d'hydrolyses avec des séquences suivantes :
amorces pour F57
| | |
|---|---|
| F57qPCRF | GCCCATTTCATCGATACCC |
| F57qPCRR | GTACCGAATGTTGTTGTCAC, |
sonde pour *F57*
| | |
|---|---|
| F57qPCRTM | FAM - CAATTCTCAGCTGCAACTCGAACACAC - BHQ, |
amorces pour IS*900*
| | |
|---|---|
| IS900qPCRF | GATGGCCGAAGGAGATTG |
| IS900qPCRR | CACAACCACCTCCGTAACC, |
sonde pour IS*900*
| | |
|---|---|
| IS900qPCRTM | FAM - ATTGGATCGCTGTGTAAGGACACGT - BHQ, |
quand la sonde pour la commande d'amplification interne a de la composition
| | |
|---|---|
| IACqPCRTM | Cy5 - GGCTCTTCTATGTTCTGACCTTGTTGGA - BHQ. |

2. Le procédé de la demande 1, **caractérisé par le fait que** la détection et la quantification de *Mycobacterium avium* sous-espèce du *paratuberculosis* sont effectuées dans les échantillons d'origine animale, dans des échantillons cliniques chez les patients humains, dans les échantillons d'origine végétales, et dans les échantillons de l'environnement, particulièrement de l'engrais, de la poussière, des foins, et dans les échantillons provenant de découvertes archéologiques ou dans des échantillons alimentaires et de fourrages.

3. Les kits de détection et du diagnostic de *Mycobacterium avium* sous-espèce du *paratuberculosis* selon la demande 1, comprenant toutes les amorces et les sondes suivantes, qui se composent en séquences mentionnées ci-dessous:
amorces pour *F57*
| | |
|---|---|
| F57qPCRF | GCCCATTTCATCGATACCC |
| F57qPCRR | GTACCGAATGTTGTTGTCAC, |
sonde pour *F57*
| | |
|---|---|
| F57qPCRTM | FAM - CAATTCTCAGCTGCAACTCGAACACAC - BHQ, |
amorces pour IS*900*
| | |
|---|---|
| IS900qPCRF | GATGGCCGAAGGAGATTG |
| IS900qPCRR | CACAACCACCTCCGTAACC, |
sonde pour IS*900*
| | |
|---|---|
| IS900qPCRTM | FAM - ATTGGATCGCTGTGTAAGGACACGT - BHQ, |
et de la sonde pour le contrôle d'amplification interne
| | |
|---|---|
| IACqPCRTM | Cy5 - GGCTCTTCTATGTTCTGACCTTGTTGGA - BHQ. |
